Europäisches Patentamt

European Patent Office

Office européen des brevets

(11) Publication number: **0 047 879**
**B1**

(12) **EUROPEAN PATENT SPECIFICATION**

(45) Date of publication of patent specification: **16.05.84**

(51) Int. Cl.³: **C 12 P 21/00, C 12 P 21/06**

(21) Application number: **81106486.4**

(22) Date of filing: **20.08.81**

(54) Process for preparing modified protein compositions.

(30) Priority: **11.09.80 JP 126422/80**

(43) Date of publication of application:
**24.03.82 Bulletin 82/12**

(45) Publication of the grant of the patent:
**16.05.84 Bulletin 84/20**

(84) Designated Contracting States:
**CH DE FR IT LI NL SE**

(56) References cited:
**DE-A-2 909 854**

**CHEMICAL ABSTRACTS, vol. 91, no. 7, 13th
August 1979, page 541, no. 54886r Columbus,
Ohio, U.S.A. M. YAMASHITA et al.: "A novel
one-step process for enzymic incorporation of
amino acids into proteins: APPLICATION TO
SOY PROTEIN AND FLOUR FOR ENHANCING
THEIR METHIONINE LEVELS"
CHEMICAL ABSTRACTS, vol. 90, no. 9, 26th
February 1979, page 407, no. 70795v
Columbus, Ohio, U.S.A.: M. YAMASHITA et al.:
"A one-step process for incorporation of L-
methionine into soy protein by treatment with
papain"**

(73) Proprietor: **TERUMO CORPORATION
44-1, 2-chome, Hatagaya Shibuya-Ku
Tokyo 151 (JP)**

(72) Inventor: **Yamazaki, Ken-ichi
13-26, 6-chome, Arajuku-machi
Kawagoe-shi Saitama-ken (JP)**
Inventor: **Takao, Shoji
10-4, 4-chome, Minamizawa
Higashikurume-shi Tokyo (JP)**
Inventor: **Yamamoto, Koji
3-14-502, 2-chome, Nijigaoka Tama-ku
Kawasaki-shi Kanagawa-ken (JP)**

(74) Representative: **Patentanwälte Henkel,
Pfenning, Feiler, Hänzel & Meinig
Möhlstrasse 37
D-8000 München 80 (DE)**

(56) References cited:
**CHEMICAL ABSTRACTS, vol. 91, no. 7, 13th
August 1979, page 542, no. 54887s Columbus,
Ohio, U.S.A. S. ARAI et al.: "A novel one-step
process for enzymic incorporation of amino
acids into proteins: papain-catalyzed
polymerization of L-methionine ethyl ester
and its regulation by adding a protein substrate"**

**Description**

Background of the invention

Field of the invention

This invention relates to a process for preparing a modified protein composition obtained by modifying the amino acid composition of a protein raw material, and more particularly, to a process for preparing a modified protein composition having an amino acid composition modified by incorporating an amino acid into a protein raw material in the form of a peptide bond through the reverse reaction of proteolysis caused by proteases acting on the protein raw material. By the term "modified protein composition" used in this specification is strictly meant a mixture of proteins and/or peptides having the intended amino acid or acids incorporated therein.

Description of the prior art

When the amino acid composition of protein material is modified in order to increase its nutritive value, it is a common practice in the art to simply add free amino acids to the protein material. However, in changing the properties of a protein raw material by tailoring its amino acid composition, partly because food containing such modified protein should be free of the taste and odor inherent to amino acids and partly because the food should be stable during storage, processing and cooking, it is rather preferably to incorporate amino acids to be enriched into the protein raw material in the form of a peptide bond.

Although proteases or proteolytic enzymes generally promote the cleavage of a peptide linkage, they tend to reverse its action toward the synthesis of a peptide bond as the substrate concentration is increased to an extremely high level. This tendency is the reverse reaction of proteolysis and is generally designated as the plastein reaction. It is known that if an amino acid ester coexists during the plastein reaction, the amino acid is also incorporated in the form of a peptide bond. See *Agric. Biol. Chem., 43,* (5), 1065—68 (1979), and *J. Agric. Food Chem., 27,* (1), 52—56 (1979). As described in these articles, amino acids may be incorporated into protein material by either of the two processes: two-step process in which protein material is first subjected to enzymatic hydrolysis and then to plastein synthesis in the presence of the intended amino acid ester, and one-step process in which protein material is subjected to an enzymatic treatment in the presence of the intended amino acid ester by means of the reaction system of so-called aminolysis. Although the latter process cannot be designated as the plastein reaction or synthetic reaction in a strict sense, the term "plastein reaction" used in this specification is intended to include both the processes. The present invention proceeds with the latter one-step process. Making researches on the commercial application of the plastein reaction with the object of modifying the amino acid composition of protein material to improve its nutritive value or to alter its properties, the inventors have succeeded in incorporating the required or intended amino acid into a variety of protein materials in the form of a peptide bond in a simple economical way while satisfying the safety requirement of food.

Free amino acids, which are inert to the reaction, must be converted into an active form, for example, an ester form before amino acids can be bonded with protein materials or peptide compounds through the plastein reaction. In incorporating an amino acid into a protein material or a peptide compound, it is the most important for commercial manufacture how to prepare an amino acid ester in an economical and safe manner. A variety of methods are known for the esterification of amino acids, including the hydrogen chloride process, sulfuric acid process, thionyl chloride process, strongly acidic cation exchange resin process and the like. Considerations have also been directed to the purification of the thus prepared esters. However, as long as the plastein reaction starts with an isolated pure amino acid ester, this reaction cannot be accomplished with any economic advantages independent of the process selected for the preparation of the ester used. This process is thus commercially unnacceptable. In addition, it should be avoided from a view point of food safety to use various chemical substances to isolate and purify the ester. For an economic supply of an ester with food safety, the process should be as simple as possible and the use of additional chemical substances should be avoided. Making further researches into this problem, the inventors have found that this object can be fully attained by selecting ethyl alcohol as the best alcohol for esterification under the requirement of food safety, using concentrated sulfuric acid which acts as a catalyst and serves to remote water formed during the reaction, and transferring the reaction solution from the esterification step to the plastein reaction step without isolating or purifying the reaction product.

Summary of the invention

An object of the present invention is to provide a process for preparing a modified protein composition in which the necessary or intended amino acid can be incorporated into a protein material or peptide compound through the plastein reaction to modify the amino acid composition of the protein material such that amino acids are in more readily absorbable form as compared with the prior art process using an isolated pure amino acid.

Briefly stated, the present invention provides a process for preparing a modified protein composition by adding a protease to a protein raw material, treating the material with water so as to

2

give a substrate concentration of 10—60 w/v%, thoroughly stirring the raw material system, adding a reaction solution obtained through the esterification of an amino acid to be incorporated in an alcohol, preferably ethyl alcohol in the presence of concentrated sulfuric acid as a catalyst to the raw material system in a sufficient amount to give the desired amount of the amino acid, and allowing enzymatic reaction to take place at a pH value suitable for the enzyme used and at a temperature of 20—70°C for 8—48 hours, thereby incorporating the intended amino acid into the protein material in the form of a peptide bond.

Problems encountered in using the amino acid ester reaction solution directly in the plastein reaction without any treatment are that the ester can be hydrolyzed back to a free amino acid during the period between the end of esterification and the start of plastein reaction, and that the alcohol has some influence on the enzyme during the plastein reaction. The water formed in the reaction system must be removed in order to prevent hydrolysis of the amino acid ester. It is effective to this end to use sulfuric acid as a catalyst for esterification because sulfuric acid is capable of taking up water. Since the esterification is effected in the presence of an extremely excessive amount of an alcohol, unreacted alcohol is also present in the enzymatic reaction solution when the ester reaction solution is used in the plastein reaction without any treatment. There is the possibility that higher concentrations of such unreacted alcohol could adversely affect the enzymatic activity. However, ethyl alcohol, for example, has only a negligible influence on common proteases provided that its concentration is lower than about 30%. It is therefore, desired to adjust conditions for the plastein reaction such that the concentration of ethyl alcohol in the reaction solution may not exceed 30%.

In order to increase the rate of incorporation of an amino acid through the plastein reaction, the reaction should be effected at a substrate concentration as high as possible. For effective reaction, the substrate concentration should be higher than 10%, more preferably higher than 15%. When a protein raw material in the form of powder is dispersed in water at a high concentration, mixing of powder with water is very difficult and undissolved lumps of powder often form. As a result, the enzymatic reaction does not fully proceed, and the rate of incorporation of an amino acid becomes low. In order to increase the amino acid incorporation rate, not only the substrate concentration should be increased, but also the substrate or protein material should be uniformly dispersed in the reaction solution. It is therefore important how to disperse the protein raw material in water. The inventors have found that the following procedure of dispersing powdery protein raw material in water is most effective to avoid formation of lumps. A lump-free homogeneous dispersion may be prepared by gradually adding protein raw material to a buffer solution having proteases dissolved with stirring until the predetermined amount of protein material has been added, and thoroughly stirring the mixture. It is believed that this favorable result is obtained because the proteases exert their liquefying action most effectively. The rate of incorporation of a desired amino acid may be increased by adding a reaction solution containing the amino acid ester to the above-prepared protein material dispersion because the combined solution can be uniformly stirred during the reaction period. Using this procedure, dispersion of the raw material and the subsequent reaction can be conducted in a common reaction vessel without the need for a special reaction vessel equipped with an agitator.

The protein raw materials which can be used in the process of the present invention include soybean protein, egg protein, milk casein, wheat gluten, corn zein, fish meal protein, yeast protein, Chlorella protein and the like. When the protein material to be used is untreated, it may preferably be pretreated in a conventional denaturation manner, for example, by a heat treatment, alcohol treatment, alkali treatment and the like, to thereby convert the protein material into a state more susceptible to enzymatic action. As long as separated or purified protein materials are used, amino acids may be fully incorporated without any additional pretreatment of the protein material.

The proteases which can be used for the amino acid incorporation include papain, pepsin, bromelain, ficin, Molsin (Seishin Pharmaceutical Co., Ltd) Nagase (Nagase & Co., Ltd.), Pronase (Kaken Chemical Co., Ltd.), Thermoase (Daiwa Kasei K. K.), Rapitase (Takeda Chemical Industries Ltd.) and the like.

The alcohols used may preferably be alcohols of food additive grade, for example, ethyl alcohol, decyl alcohol and benzyl alcohol because the retention of unreacted alcohol must be taken into account. The most preferred is ethyl alcohol.

Since the modified protein composition obtained by incorporating a particular amino acid therein by the process of the present invention contains the unreacted amino acid ester and inorganic salt, it is desired to remove such impurities depending on the intended use of the composition. Since the majority of the products resulting from the plastein reaction are insoluble in water, the unreacted reagents and inorganic salts may be removed by subjecting the neutralized reaction solution to centrifugal separation or filtration followed by rinsing. To recover the product in high yields, a membrane-using treatment such as dialysis and ultrafiltration is useful. The thus treated modified protein composition, after concentrated if necessary, may be dried in a conventional manner as by spray drying or freeze drying into a finished product.

The process of the present invention may be adapted to a variety of applications. It is generally known that a protein raw material contains its own limiting amino acid. If such a limiting amino acid is desired to be enriched, an ester of the limiting amino acid may be used. If it is desired to enrich another particular amino acid to give rise to an intensional amino acid imbalance, then an ester of this particular amino acid may be used. Furthermore, if the resulting protein composition should be solubilized, a water-soluble amino acid may be incorporated in the form of a peptide bond.

## Description of the preferred embodiments

The present invention will be more fully understood from the description of preferred examples. A comparison of amino acid utilization rate is also made between the present process and the prior art process.

## Example I

0.67 g of papain and 50 mg of L-cystein as an activator for the papain were dissolved in 52 ml of water containing 0.52 g of sodium carbonate and 1.72 g of sodium hydrogen carbonate, and then with stirring 20 g of a commercially available soybean protein isolate was added to the solution and thoroughly mixed therewith. Separately, 0.82 ml of concentrated sulfuric acid was gradually added to a suspension of 1 g of L-methionine in 7 ml of ethyl alcohol until the L-methionine was thoroughly dissolved, and then the solution was heated under reflux at 80°C for 3 hours, obtaining an ester reaction solution having a degree of esterification of 92.7%. This reaction solution was added to and thoroughly mixed with the above-prepared protein raw material system, and then enzymatic reaction was allowed to take place at 40°C for 24 hours. The thus obtained product had an L-methionine incorporation rate of 88.4%. After water was added to the reaction solution to give a total volume of 500 ml, the solution was subjected to dialysis in water at 5°C for 72 hours using a cellulose tube to thereby remove inorganic salts and the unreacted ethyl L-methionine and then freeze dried, obtaining 17.4 g of a modified protein composition in which L-methionine is incorporated in the form of a peptide bond. An analysis by an amino acid analyzer showed that neither free amino acids nor ethyl L-methionine was present in the protein composition.

The protein composition having methionine incorporated therein by the above process is modified in amino acid composition as compared with the starting soybean protein material which contains methionine as the primary limiting amino acid as shown in Table I.

### TABLE I

unit: % by weight

| Amino acid | Soybean protein isolate | Methionine-incorporated product |
|---|---|---|
| Aspartic acid | 12.1 | 11.5 |
| Threonine | 3.7 | 3.8 |
| Serine | 4.8 | 4.6 |
| Glutamic acid | 20.4 | 19.2 |
| Proline | 2.7 | 2.5 |
| Glycine | 4.1 | 4.1 |
| Alanine | 4.2 | 4.2 |
| Cysteine | 0.9 | 0.7 |
| Valine | 5.3 | 5.0 |
| Methionine | 1.3 | 4.7 |
| Isoleucine | 5.5 | 5.1 |
| Leucine | 8.1 | 7.8 |
| Tyrosine | 4.2 | 4.0 |
| Phenylalanine | 5.2 | 5.3 |
| Lysine | 5.7 | 5.5 |
| Histidine | 2.6 | 3.5 |
| Arginine | 7.7 | 7.4 |
| Tryptophane | 1.5 | 1.1 |

## Example II

3.5 ml of ethyl alcohol and 0.5 ml of sulfuric acid were added to 0.6 g of L-lysine and heated under reflux at 80°C for 3 hours, obtaining ethyl L-lysine in a yield of 92.8%. 150 mg of bromelain and 25 mg of L-cystein were dissolved in 15 ml of water containing 0.15 g of sodium carbonate and 0.4 g of sodium hydrogencarbonate, then 5 g wheat gluten (Wako Pure Chemical Industries Ltd.) as a raw material was added little by little to the solution and agitation was performed for mixing. The total volume of the above-prepared ethyl L-lysine reaction solution was added to the raw material system, which was incubated at 37°C for 30 hours. After completion of the reaction, water was added to the solution to give a total volume of 400 ml and the solution was neutralized with aqueous hydrochloric acid and then subjected to centrifugal separation under 8000×G to separate the insoluble fraction. The supernatant was subjected to ultrafiltration using a ultrafilter having a fractional molecular weight of 1000 (Amicon UM-2) to remove the low-molecular weight impurities. The resulting liquor was combined with the centrifuged precipitate and freeze dried, obtaining 4.3 g of a modified protein composition having L-lysine enriched. The protein composition having L-lysine incorporated therein is modified in amino acid constitution as compared with the wheat gluten which contains lysine as the limiting amino acid, as shown in Table II.

TABLE II

| Amino acid | Wheat gluten | unit: % by weight Lysine-incorporated product |
|---|---|---|
| Aspartic acid | 4.4 | 4.1 |
| Threonine | 2.8 | 2.6 |
| Serine | 4.8 | 4.4 |
| Glutamic acid | 31.9 | 30.4 |
| Proline | 12.1 | 11.7 |
| Glycine | 3.5 | 3.2 |
| Alanine | 2.4 | 2.2 |
| Cysteine | 3.6 | 3.4 |
| Valine | 4.6 | 4.3 |
| Methionine | 1.7 | 1.6 |
| Isoleucine | 4.1 | 3.8 |
| Leucine | 7.2 | 6.8 |
| Tyrosine | 3.1 | 3.0 |
| Phenylalanine | 4.7 | 4.5 |
| Lysine | 2.3 | 7.6 |
| Histidine | 1.8 | 1.6 |
| Arginine | 3.9 | 3.8 |
| Tryptophane | 1.1 | 1.0 |

The above examples reveal that a particular amino acid can be effectively incorporated into a protein raw material by way of the plastein reaction according to the present invention. The conventional process, on the other hand, uses a particular amino acid ester in an isolated, purified form. Therefore, the conventional process needs extra steps for isolation and purification and as a result, the amino acid ester is too expensive to commercialize the process. Instead of using an isolated pure amino acid ester, the process of the present invention uses an ester reaction solution containing a sulfuric acid catalyst in incorporating an amino acid by way of the plastein reaction. The amino acid to be incorporated is more effectively utilized in the present process than in the conventional process using an isolated pure amino acid ester. This will be demonstrated by referring to the following comparative examples. In these comparative examples, L-methionine was introduced into soybean protein material in the same manner as in Example I except that L-methionine esters were prepapred by the three different methods shown below and added to the enzymatic reaction solution. The results of Example I according to the present invention are shown in Table III together with the results of the comparative examples.

Comparative Example I

7 ml of ethyl alcohol was added to 1 g of dry finely divided L-methionine to form a suspension. With stirring at a temperature of 30—40°C, 10 millimol of hydrogen chloride gas was introduced into the suspension. The suspension was heated uder reflux for 3 hours. The reaction solution was cooled and subjected to vacuum distillation at a temperature of lower than 30°C to expel the ethanol. The precipitated ethyl L-methionine hydrochloric acid was recrystallized from ether and the resulting crystals were dried.

Comparative Example II

L-methionine was esterified in the presence of a sulfuric acid catalyst as in Example I. The reaction solution was cooled to below 10°C and neutralized with ice-cooled ammonia. The precipitated crystals were collected by filtration, washed with ethanol, and dried in vacuum.

Comparative Example III

An ethyl L-methionine reaction solution obtained by the same esterification process as in Comparative Example I was directly used in enzymatic reaction.

# 0 047 879

TABLE III

| | Example | Comparative Example | | |
|---|---|---|---|---|
| | I | I | II | III |
| Ester yield | 92.7 | 78.2 | 75.0 | 92.5 |
| Incorporation rate | 88.4 | 79.4 | 83.1 | 78.2 |
| Methionine utilization | 81.9 | 62.1 | 62.3 | 72.3 |

As apparent from the above-described Examples and Comparative Examples and Table III, the process of the present invention allows a particular amino acid to be incorporated in a simple manner because it is a one-step process and remarkably increases the rates of incorporation and utilization of the particular amino acid so that it may be effectively applied as a commercial process.

**Claims**

1. A process for preparing a modified protein composition, comprising the steps of:

dissolving a protease in an aqueous buffer solution which has been adjusted to an optimum pH, adding a protein raw material to the solution to a concentration of 10 to 60 w/v% with stirring, continuing stirring until a homogeneous dispersion is obtained, esterifying an amino acid in an alcohol in the presence of sulfuric acid as a catalyst to form an amino acid ester reaction solution, adding the amino acid ester reaction solution to the dispersion, and allowing enzymatic reaction to take place in the reaction mixture, thereby causing the amino acid to bond with the protein raw material in the form of a peptide bond.

2. A process according to claim 1 wherein said alcohol is an alcohol of food additive grade.
3. A process according to claim 2 wherein said alcohol is ethyl alcohol.
4. A process according to any one of claims 1—3 wherein said protease is selected from the group consisting of bromelain and papain.
5. A process according to any one of claims 1—3 wherein said protein raw material is soybean protein and said amino acid is methionine.
6. A process according to any one of claims 1—3 wherein said protein raw material is wheat gluten and said amino acid is lysine.

**Revendications**

1. Procédé pour préparer des compositions protéiniques modifiées, comprenant les étapes suivantes:

— dissolvant une protéase dans une solution aqueuse tampon ajusté à un pH optimal,
— ajoutant sous agitation une matière protéinique brute à la solution jusqu'à une concentration comprise entre 10 et 60% en poids/volume,
— continuant l'agitation jusqu'à l'obtention d'une dispersion homogène,
— estérifiant un acide aminé au sein d'un alcool en présence d'acide sulfurique comme catalyseur, pour obtenir une solution réactionnelle d'ester d'acide aminé,
— ajoutant la solution réactionnelle d'ester d'acide aminé à la dispersion, et
— permettant le déroulement d'une réaction enzymatique dans le mélange réactionnel, provoquant la liaison de l'acide aminé à la matière protéinique brute sous forme d'une liaison peptidique.

2. Procédé selon la revendication 1 où le dit alcool a une pureté alimentaire.
3. Procédé selon la revendication 2 où le dit alcool est l'alcool éthyique.
4. Procédé selon l'une quelconque des revendications 1 à 3 où la dite protéase est choisie parmi la bromélaïne et la papaïne.
5. Procédé selon l'une quelconque des revendications 1 à 3 où la dite matière protéinique brute est la protéine de soja, et le dit acide aminé est la méthionine.
6. Procédé selon l'une quelconque des revendications 1 à 3 où la dite matière protéinique brute est le glute de blé, et le dit acide aminé est la lysine.

**Patentansprüche**

1. Verfahren zur Herstellung einer modifizierten Proteinmasse, dadurch gekennzeichnet, daß man eine Protease in einer auf einen optimalen pH-Wert eingestellten wäßrigen Pufferlösung löst, die Lösung unter Rühren bis zu einer Konzentration von 10—60 Gew.-% (w/v%) mit einem Proteinrohmaterial versetzt, so lange weiterrührt, bis eine homogene Dispersion erhalten wird, eine Aminosäure in einem Alkohol in Gegenwart von Schwefelsäure als Katalysator zur Bildung einer Aminosäureesterreaktionslösung verestert, die Aminosäureesterreaktionslösung zu der Dispersion hinzufügt und in dem Reaktionsgemisch eine enzymatische Reaktion ablaufen.läßt, wobei die Aminosäure mit dem Proteinrohmaterial in Form einer Peptidbindung eine Bindung eingehen gelassen wird.

2. Verfahren nach Anspruch 1, dadurch gekennzeichnet, daß der Alkohol aus einem Alkohol eines für Nahrungsmittelzwecke geeigneten Reinheitsgrads besteht.

3. Verfahren nach Anspruch 2, dadurch gekennzeichnet, daß der Alkohol aus Ethanol besteht.

4. Verfahren nach einem der Ansprüche 1 bis 3, dadurch gekennzeichnet, daß als Protease Bromelain oder Papain verwendet wird.

5. Verfahren nach einem der Ansprüche 1 bis 3, dadurch gekennzeichnet, daß das Proteinrohmaterial aus Sojabohnenprotein und die Aminosäure aus Methionin bestehen.

6. Verfahren nach einem der Ansprüche 1 bis 3, dadurch gekennzeichnet, daß das Proteinrohmaterial aus Weizengluten und die Aminosäure aus Lysin bestehen.